Europäisches Patentamt

European Patent Office

Office européen des brevets

(11) Publication number : **0 549 002 A1**

# (12) EUROPEAN PATENT APPLICATION

(21) Application number : **92202389.0**

(22) Date of filing : **03.08.92**

(51) Int. Cl.⁵ : **A61K 37/02, // (A61K37/02, 31:71)**

(30) Priority : **15.08.91 NL 9101390**

(43) Date of publication of application :
**30.06.93 Bulletin 93/26**

(84) Designated Contracting States :
**AT BE CH DE DK ES FR GB GR IT LI LU MC NL PT SE**

(71) Applicant : **Hak, Barend Willem**
**Hak Pharma B.V., Leemansstraat 2**
**NL-4250 Werkendam (NL)**

(72) Inventor : **Hak, Barend Willem**
**Hak Pharma B.V., Leemansstraat 2**
**NL-4250 Werkendam (NL)**

(74) Representative : **Siemens, Andreas Meinhard Ernest, Dipl.-Ing.**
**SIEMENS & CIE. Roskam 8**
**NL-4813 GZ Breda (NL)**

(54) **Method for the preparation of an antiseptic composition containing clindamycin-hydrochloride and polymyxin.**

(57)   A composition of antibiotics, to be used as an antiseptic against gram-negative bacteria and other mycoplasms, containing clindamycin-hydrochloride and polymyx in sulphate.

Microphot.1

Microphot.2

A method for the preparation of a composition with antiseptic properties.

The present invention is relating to a method for the preparation of a substance having marked antiseptic properties. It can be used as a disinfectant and bactericidal agent for human and veterinary applications. According to the invention newly discovered properties of two antibiotic compounds are combined.

The composition according to the present invention is a combination of the two basic antibiotic substances Clindamycin-hydrochloride and Polymyxin E (Colistin-sulphate) in pure sterilized water for parenteral administration.

Clindamycin is methyl-7-chloro-6,7,8-tri-desoxy-6-trans-(1-methyl-4-propyl-L-pyrrolidine-2-carboxamido)-1-thio-L-treo-α-D-galacto-octa-pyranoside,
or: 7-chloro-7-desoxy-lincomycine (Formula I).

Polymyxin E (Colistin) is a basic cyclic polypeptide, produced by Aerobacillus Colistinus (Formula II).

In U.S.Patent 4,510,132 and in Antimicrob.Agents Chemoter. 8,(1975), 164, antimicrobial effects of Clindamycin with Colistin have been described, however only against Eschericia Coli.

The applicant investigated activity against other micro-organisms, and toxicity in mammals, as well as concentration-time after intra-muscular injections.

By this research additional characteristics of a combination of Clindamycin with Polymyxin E (Colistin) have been found.

The polymyxins are basic substances produced by the growth of strains of Bacillus Polymyxa (or Bacillus Aerosporus). The molecular weight of these cyclic polypeptides is about 1000, and with acids soluble salts can be produced.

Polymyxins have a bactericidal activity towards several gram-negative bacilli, like Pseudomonas Aeruginosa, Eschericia Coli and Salmonellae. This activity is primarily characterized by combining to the bacterial cytoplasmic membrane and changing the permeability of same.

Generally, gram-negative bacteria, but also many other micro-organisms, possess an extra-ordinary efficient barrier against many external influences, especially the outer membrane which is a unique structure located outside the peptidoglycan. Said membrane renders to these organisms a resistance to any host defence factors, such as lysozyme. The outer membrane also excludes or reduces significantly the penetration of several antibiotic substances.

The polymyxins as a polycationic amphipathic group of antibiotics are bactericidal to gram-negative bacteria by virtue of a dual mechanism of activity. They disorganize the outer membrane and penetrate to reach their final bactericidal target, the cytoplasmic membrane.

It has been found by applicant that the dis-organization of the outer membrane by a polymyxin, and also by a polymyxin from which the fatty acid tail was removed, sensitizes gram-negative bacteria to antibacterial agents and to the host-defence mechanisms complementary, and makes these bacteria susceptible to certain antibiotics, and even a marked synergism has been found.

Furthermore a safely and easily administratable form of curative with both active ingredients has now been developed.

It has been found, that the activity of Clindamycin is very much intensified in combination with Polymyxin E, and not only towards Eschericia and Salmonellae but also towards Pasteurella and several Mycoplasms (Microphotos 1 and 2).

The medicament is made by mixing the polymyxin as sulphate with Clindamycin-hydrochloride in a ratio of 10-80% wt./wt. of Polymyxin E and 80-10% wt./wt. of Clindamycin with 10% wt./wt. of pure sterile water, with a slight dose of emulsifier if necessary.

The pure polymyxin is not very stable. It appeared to be stabilized by Clindamycin and the acute toxicity for mammals and humans was strikingly reduced.

The properties of the novel composition and the applications are apparent from the following experimental series:

1: Treatment of infections:

Pasteurella spp. can infect the respiratory tract of humans and animals and antimicrobial agents are used to treat these infections.

Two clinical isolates of Pasteurella haemolytica were studied. The bacteria were isolated from calves and identified. The isolates included P. haemolytica 3R188 and 6R132. Minimal inhibitory concentrations of the antimicrobial combinations for the clinical isolates were determined by a plate dilution method as described by Bactolab B.V. Test concentrations of clindamycin (as clindamycin hydrochloride) and colistin sulphate were chosen as 0,05, 0,5 and 5,0 g/ml, separately and in combination.

Stock solutions containing 400 μg/ml of clindamycin and colistin were made in sterile purified water; dilutions containing 200, 20 and 2 $\mu$g/ml were prepared by further dilution. To 1 ml of the individual antibiotic solutions or to twice 0,5 ml of the solutions, 19 ml of fluid (5%) horseblood-agar was added. The mixture was stirred and the agar transferred into sterile petri-dishes. The plates were incubated with a multipoint inoculator of Steers. The strength of the inoculating suspension was 2 MacFarland. The plates were observed during 24 hours after incubation.

The minimal inhibitory concentrations (MICs) of both P. haemolytica were 1,6 $\mu$g/ml for colistin and >20 $\mu$g/ml for clindamycin. In combination, the MIC was reached at a final concentration of 5,0/0,5 $\mu$g/ml (clindamycin/colistin). At a final concentration of 5/0,05 also some inhibition was noticed.

## 2. Decrease of minimal inhibitory concentration:

Mycoplasmas can infect the respiratory tract of humans and animals. Antimicrobial agents are used to treat these infections.

For the investigation, type strain BTS-7 of Mycoplasma hyorhinis was used of the Institute of Medical Microbiology, Aarhus, (Denmark). The strain was investigated by the serial broth dilution method. Both clindamycin (as clindamycin-hydrochloride) and colistin-sulphate were serially diluted twofold in 10 wells of a microtiter plate. Friis-broth was added to each well. A standard number of organisms ($10^3$x $10^5$ color-changing units (CCU) per ml)grown in Friis broth was used. An amount of 175 $\mu$l, containing $1.7\text{x}10^2$ to $1.7\text{x}10^4$ CCU, was added to each well. The media contained no other bacterial inhibitors, with exception of the combination of clindamycin/colistin.

The susceptibility or resistance of the strain to the antimicrobial agent(s) was indicated by whether the culture was able to metabolize glucose in the presence of one (or both) of the concentrations of the antimicrobial agent(s). Glucose metabolism was visualized by a change of the phenol-red indicator from red to yellow (Taylor-Robinson, Methods in Mycoplasmology I, Acad. Press. Inc., 411,1983). On the day of inoculation, the CCU was prepared from frozen stock cultures with a known number of cells; organisms were counted again to verify the actual number of organisms in the system. The microtiter plates were sealed with an adhesive tape and incubated aerobically at 37°C. The minimal inhibitory concentration (MIC) of the antimicrobial agent(s) was determined as the lowest concentration at which the medium did not change colour. The MICs for M. hyorhinis were recorded when colour changes had stopped for two days (day 5).

The MIC found for clindamycin alone was 1.0 $\mu$g/ml. For colistin, no actual MIC was found (>50 $\mu$g/ml).

In combination, the MIC for clindamycin decreased in the presence of colistin sulphate (concentration 0.2/0,2 $\mu$g/ml, clindamycin/colistin).

## 3. Toxicology:

As demonstrated with individual toxicity data of clindamycin (as hydrochloride or phosphate-salt) and colistin sulphate, the latter substance alone has most serious toxicological properties.

Clindamycin principally does not possess harmful toxicological properties except, if any, hypersensitivity reactions. Since the toxicological signs and sites of action of clindamycin and colistin-sulphate differ so much from each other, it is concluded that the combination of clindamycin and colistin-sulphate renders a decrease in toxicity.

This statement was confirmed with tolerance tests of the present product. In this study the normal therapeutical dose of 1 ml injectable solution (an aqueous solution containing per ml 100 mg clindamycin (as clindamycin-hydrochloride) and 200.000 IU colistin-sulphate) per 10 kp of bodyweight per day was given intramuscularly (into the dorsal region of the neck, left and right side alternately) to piglets (bodyweight 10-12 kg) for 5 consecutive days. Additionally, five times the therapeutical dose (i.e. 5 ml per 10 kg bodyweight per day) was given to another group of piglets for 5 consecutive days. The application period of 5 days was followed by 10 days of subsequent observation and the results from the test groups were compared with those of a comparison group from which animals received 1,0 ml or 5,0 ml of physiologic saline solution per 10 kg of bodyweight, respectively.

Based on this test program, the following results were reported:

(1) The general state of health, food intake, defecation and urination, visible micosi and body temperature did not indicate any influence after administration of the product-composition.

(2) The administration of the present product did not influence the development of the pigs' bodyweight negatively.

(3) The haematological analysis proved that the present product did not change the haemotological status of the animals (i.e. haematicrit, erythrocytes, thrombocytes, leucocytes, haemoglobin concentrations and differential blood-cell count).

(4) Clinico-chemical analysis did not show any effect of the administration of the product, except with regard to aspartate-aminotransferase. The intensification of the aspartate-aminotransferase activity resulting from the administration of the composed product are considered to be physiological metabolic responses of the liver.

(5) The genotoxicity tests proved,that the present product did not induce any chromosome aberrations in the peripheral blood-lymphocytes of pigs.

(6) The pathological-histological findings as well as the organ weights, observed at slaughter 12 days after the last administration of the product, corresponded to physiological conditions. No influences of the product were detectable.

(7) The present product caused local responses (swellings and hardenings), particularly at the dosage of 5,0 ml per 10 kg of bodyweight, which can be described as oedematous soakings.

Only insignificant oedematous soakings are to be expected at the therapeutical dose of 1,0 ml per 10 kg bodyweight. The local responses declined within 5 to 7 days after the end of application. This local response was also confirmed with residue investigations performed with the present product (injectable solution) in pigs, calves and cows,after filing the priority application.

In these studies complete and fast absorption of the compounds was demonstrated and only some induced irritation and tissue damage occured at the injection site(s). Again restored impressions were observed within 14 days after last administration.

4. Synergistic activity:

The therapeutical use of the combination was investigated to prove the synergistic action of the combination clindamycin with colistin. Based on the pharmacokinetic parameters obtained with pharmacokinetic investigations of the injectable solution in pigs, calves and cows,at a daily dosage of 1 ml of injectable solution per 10 kg of bodyweight intramuscularly, it appeared to be necessary to administer this dosage twice daily for beneficial therapeutic response. The pharmacokinetic parameters in cows and pigs are showed in the following tables:

Table 1. Area under the plasma concentration-time curves (AUC), maximum plasma concentrations (Cmax), time of Cmax (Tmax) after three intramuscular injections:

Clindamycin in cows:

| Cow no.: | AUC 0-24 hr mg.h/l | Cmax* (mg/l) | Tmax* (hrs) | Elimination half-life (hrs) first injection | last |
|---|---|---|---|---|---|
| 37 | 12.58 | 0.80 | 0.5 | 16.0 | 9.6 |
| 56 | 10.25 | 0.74 | 1.0 | 12.9 | 14.5 |
| 57 | 15.33 | 1.28 | 0.5 | 7.4 | 9.9 |
| 60 | 10.85 | 1.32 | 0.5 | 9.4 | 9.3 |
| mean | 12.25 | 1.04 | | 11.4 | 10.8 |
| s.d. | 2.28 | 0.31 | | 3.8 | 2.5 |

Colistin in cows:

| Cow no.: | AUC 0-24 hr mg.h/l | Cmax* (mg/l) | Tmax* (hrs) | Elimination half-life (hrs) first injection | last |
|---|---|---|---|---|---|
| 37 | 2.40 | 0.29 | 0.5 | 21.5 | 20.7 |
| 56 | 1.54 | 0.20 | 0.5 | 21.3 | 32.4 |
| 57 | 3.07 | 0.33 | 0.5 | 18.7 | 24.0 |
| 60 | 2.94 | 0.42 | 0.5 | 31.9 | 21.4 |
| mean | 2.49 | | 3.33 | 23.4 | 24.6 |
| s.d. | 0.70 | | 2.34 | 5.8 | 5.0 |

Table 2. Area under the plasma concentration-time curves (AUC), maximum plasma concentrations (Cmax), time of Cmax (Tmax) after three intramuscular injections.

Clindamycin in pigs:

| Pig no. | Auc 0-24 hr mg.h/l | Cmax* (mg/l) | Tmax* (hrs) | Elimination half-life (hrs) 1st. inject. | 3rd. inject. |
|---|---|---|---|---|---|
| 09 | 7.28 | 1.27 | 0.5 | 5.5 | 7.5 |
| 10 | 10.28 | 1.02 | 1.0 | 5.2 | 4.9 |
| 11 | 13.87 | 1.20 | 0.5 | 6.5 | 9.3 |
| 12 | 9.45 | 1.36 | 1.0 | 6.3 | 6.1 |
| 13 | 8.32 | 1.55 | 0.5 | 5.8 | 11.1 |
| 14 | 8.26 | 1.17 | 2.0 | 6.1 | 6.5 |
| mean | 9.58 | 1.26 | 0.92 | 5.9 | 7.6 |
| s.d. | 2.35 | 0.18 | 0.58 | 0.5 | 2.5 |

Colistin in pigs:

| Pig.no.: | AUC 0-24 hr mg.h./l | Cmax* (mg/l) | Tmax* (hrs) | Elimination Half-life (hrs) 1st inject. | 3rd inject. |
|---|---|---|---|---|---|
| 09 | 1.55 | 0.15 | 0.5 | 14.3 | 22.9 |
| 10 | 3.02 | 0.22 | 2.0 | 14.2 | 17.1 |
| 11 | 1.99 | 0.25 | 0.5 | 21.3 | 15.2 |
| 12 | 1.83 | 0.21 | 1.0 | 21.5 | 10.8 |
| 13 | 3.87 | 0.50 | 0.5 | 19.7 | 18.1 |
| 14 | 2.00 | 0.20 | 1.0 | 11.1 | 15.4 |
| mean | 2.38 | 0.25 | 0.92 | 17.0 | 16.6 |
| s.d. | 0.89 | 0.12 | 0.58 | 4.4 | 4.0 |

In order to prove the synergistic action, contrary to predicted by pharmacokinetic data, clinical tests were performed with the present product (injectable solution) following daily intramuscular injections of 1 ml per 10 kg body weight for 3 consecutive days supported by the additional clinical data.

The present product was used under field conditions in cattle (333 treated animals), pigs (153 treated pigs and about 150 treated piglets), and sheep (245 treated) for the treatment of a variety of infectious diseases. The present product was given at a dosage of 1 ml per 10 kg body weight daily, 3 consecutive days. The overall results were generally very good, as summarized in the figures hereafter.

Table 3. Clinical trials with the present product in the target animal calves showed the following results in the treatment of bronchopneumonia/pleuropneumonia, caused by mixed bacterial infections:

| Trial no. | No. of calves | Indication/ pathogen. | Result: |
|---|---|---|---|
| 21 | 4 | Pasteurella, Salmonella dublin | good |
| 22 | 8 | Pasteurella, Mycoplasma spp. | good |
| 23 | 3 | Pasteurella, Salmonella, Streptococcus | good |
| 24 | 5 | Fusobacterium | good |
| 28 | 3 | Mycoplasma spp. | good |
| 29 | 10 | Pasteurella,mycoplasma spp. | good |
| 30 | 10 | Pasteurella,Salmonella,streptococcus | good |
| 31 | 2 | Fusobacterium | good |

Clinical trials with the present product in the target animal; pigs showed the following results in the treatment of rhinitis/bronchopneumonia caused by mixed bacterial infections:

| Trial no. | No. of pigs | Indication/ pathogen. | Result: |
|---|---|---|---|
| 1 | 10 | Pasteurella multocida | good |
| 2 | 3 | Haemophilus pleuropneumoniae | good |
| 3 | 7 | Pasteurella multocida,Mycoplasma spp. | good |
| 9 | 150 | Atrophic rhinitis/Pasteurella multocida | good |
| 10 | 6 | Haemophilus pleuropneumoniae | good |
| 11 | 10 | Haemophilus pleuropneumoniae | good |

Clinical trials with the present product in the target animal; pigs showed the following results in the treatment of infectious arthritis caused by mixed infections:

| Trial no. | No.of pigs | Indication/ pathogen: | Result: |
|---|---|---|---|
| 6 | 8 | Mycoplasma spp. | good |
| 7 | 2 | Streptococcus spp. | good |
| 8 | 1 | Mycoplasma spp. | good |
| 20 | 3 | Mycoplasma spp.Streptococcus spp. | good |

Clinical trials with the present product in the target animal, sheep showed the following results in the treatment of foot rot:

| Trial no. | No.of sheep | Indication/ pathogen; | Result: |
|---|---|---|---|
| 32 | 12 | Fusobacterium necrophorum | good |
| 33 | 143 | Fusobacterium necrophorum | good |
| 34 | 62 | Fusobacterium necrophorum | good |
| 35 | 28 | Fusobacterium necrophorum | good |

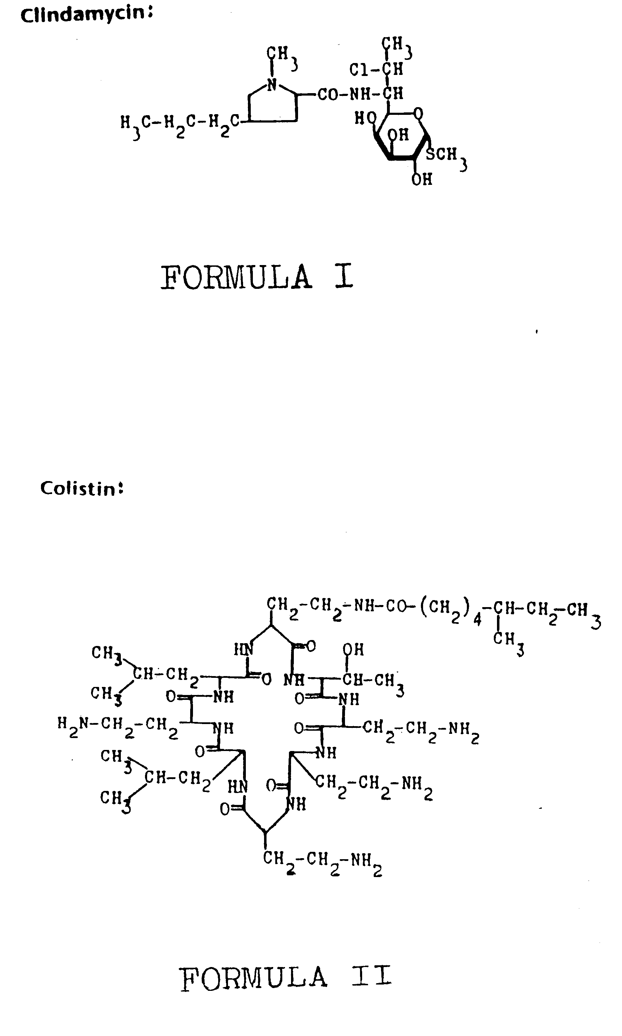

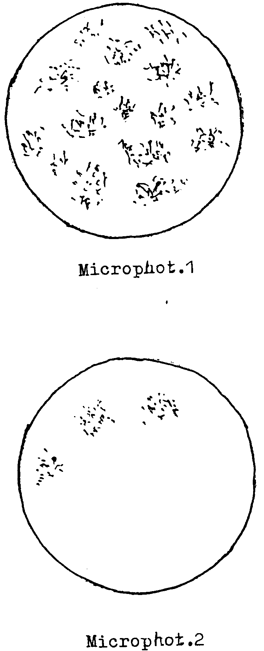

## Claims

1. A method for the preparation of a composition with antiseptic properties by a combination of basic antibiotic substances, characterized in that clindamycin-hydrochloride and polymyxinsulphate are homogeneously mixed and dissolved in pure sterilized water.

2. A method according to claim 1, characterized in that the polymyxin is polymyxin E and the ratio of the components is 10-80% wt./wt. of polymyxin E and 80-10% wt./wt. of Clindamycin.

3. A method according to claim 1, characterized in that a very small amount of a neutral soluble emulsifier is added.

4. A method according to claims 1-3, characterized in that the mixture is poured into vials for use as a parenterally administratable medicament.

5. A product prepared according to one of the preceding claims, to be used against gram-negative bacteria and other mycoplasms,which cause inflammations.

**Clindamycin:**

# FORMULA I

**Colistin:**

# FORMULA II

Microphot.1

Microphot.2

**European Patent Office**

# EUROPEAN SEARCH REPORT

Application Number

EP 92 20 2389

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CHEMICAL ABSTRACTS, vol. 86, 1977, Columbus, Ohio, US; abstract no. 13149q, LENG B ET AL. 'Susceptibilities of gram-negative bacteria to combinatios of antimicrobial agents in vitro' page 100 ;column 1 ; * abstract * & ANTIMICROB. AGENTS CHEMOTHER. vol. 8, no. 2, 1975, pages 164 - 171 | 1-5 | A61K37/02 //(A61K37/02,31: 71) |
| Y,D | US-A-4 510 132 (MARTTI VAARA) 9 March 1985 * examples 4,7 * | 1-5 | |
| Y | JAMES E.F, REYNOLDS 'Martindale, The extra pharmacopoeia' , THE PHARMACEUTICAL PRESS , LONDON * page 107, column 3, line 81 - page 108, column 1, line 6 * | 1-5 | |

TECHNICAL FIELDS SEARCHED (Int. Cl.5 )

A61K

The present search report has been drawn up for all claims

| Place of search | Date of completion of the search | Examiner |
|---|---|---|
| THE HAGUE | 26 NOVEMBER 1992 | LEHERTE C.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)